Europäisches Patentamt

European Patent Office

Office européen des brevets

(19)

(11) Veröffentlichungsnummer : **0 210 131**
**B1**

(12)                  **EUROPÄISCHE PATENTSCHRIFT**

(45) Veröffentlichungstag der Patentschrift :
07.06.89

(21) Anmeldenummer : 86810311.0

(22) Anmeldetag : 14.07.86

(51) Int. Cl.⁴ : **C 07 C 69/732, C 07 C 67/52**

(54) Lösungsmittelfreie Kristallisation von Pentaerythrit-tetrakis-[3-(3,5-di-tert.-butyl-4-hydroxyphenyl)-propionat] und dessen neue lambda-Kristallform.

(30) Priorität : 19.07.85 CH 3146/85

(43) Veröffentlichungstag der Anmeldung :
28.01.87 Patentblatt 87/05

(45) Bekanntmachung des Hinweises auf die Patenterteilung : 07.06.89 Patentblatt 89/23

(84) Benannte Vertragsstaaten :
CH DE FR GB IT LI

(56) Entgegenhaltungen :
EP--A-- 0 102 920
EP--A-- 0 148 729
JP--A--59 104 348
Die Akte enthält technische Angaben, die nach dem
Eingang der Anmeldung eingereicht wurden und die
nicht in dieser Patentschrift enthalten sind.

(73) Patentinhaber : CIBA-GEIGY AG
Klybeckstrasse 141
CH-4002 Basel (CH)

(72) Erfinder : Orban, Ivan, Dr.
Lehenmattstrasse 216
CH-4052 Basel (CH)
Erfinder : Fussenegger, Werner
Fasanenstrasse 150/5
CH-4058 Basel (CH)

## Beschreibung

Die vorliegende Erfindung betrifft ein Verfahren zur vollständigen lösungsmittelfreien Kristallisation von Pentaerythrit-tetrakis-[3-(3,5-di-tert.-butyl-4-hydroxyphenyl)-propionat] und eine neue Kristallform (λ-Modifikation) dieser Verbindung.

Bisher musste das nach an sich bekannten Methoden, wie z. B. in den US-A-3 644 482, 4 228 297 und 4 405 807 beschrieben, hergestellte und als ausgezeichnetes Antioxidans für organisches Material längst bekannte und im Handel erhältliche Pentaerythrit-tetrakis-[3-(3,5-di-tert.-butyl-4-hydroxyphenyl)-propionat] aus einem organischen Lösungsmittel umkristallisiert werden, um in einer den Erfordernissen der Technik genügenden reinen kristallinen Form erhalten zu werden. In der EP-A-102 920 ist ein allgemeines Verfahren zur Herstellung von Estern der 3-(3,5-di-tert.-butyl-4-hydroxyphenyl)-propionsäure beschrieben.

Es ist auch bekannt, dass man durch Umesterung von Methyl-3-(3,5-di-tert.-butyl-4-hydroxyphenyl)-propionat mit Pentaerythrit in Gegenwart katalytischer Mengen eines Oxids oder einer metallorganischen Verbindung von Metallen der vierten Haupt- oder Nebengruppe des Periodensystems und anschliessendes Andestillieren der erhaltenen Schmelze in einer Kurzzeit-Destillationsapparatur im Vakuum und bei hoher Temperatur, also ohne Einsatz von Lösungsmitteln, praktisch quantitativ reines Pentaerythrit-tetrakis-[3-(3,5-di-tert.-butyl-4-hydroxyphenyl)-propionat] erhält. Die nach dem Abkühlen erstarrte Produktschmelze wird zum Schluss zerkleinert. Dieses Produkt ist allerdings amorph und schmilzt bei 55-62 °C. Schon aus Gründen der Lagerungs- und Transportstabilität ist jedoch eine kristalline Struktur mit höherem Schmelzpunkt gegenüber der amorphen Form von grossem Vorteil.

Die Herstellung einer amorphen, niedrig schmelzenden (ca. 49 °C) Form der Titelverbindung ist auch in der JP-A-59-104 348 beschrieben. Sie wird durch Eintropfen der Schmelze in Wasser oder in ein inertes Gas (wobei das Einbringen mittels eines Extruders erfolgen kann) oder durch Auftropfen auf eine feste Oberfläche erhalten.

Aus technisch-wirtschaftlichen Gründen, vor allem aber aus ökologischen Gründen, wäre eine Kristallisation des Produktes ohne Verwendung eines Lösungsmittels wünschenswert. Eine Möglichkeit, die Verwendung von Lösungsmitteln zu vermeiden, besteht in der Anwendung der Schmelzkristallisation. Eine solche ist bisher für das genannte Produkt nicht bekannt. Versuche in dieser Richtung schlugen bisher fehl, da die Kristallisationstendenz der Schmelze von Pentaerythrit-tetrakis-[3-(3,5-di-tert.-butyl-4-hydroxyphenyl)-propionat] ausserordentlich schlecht ist. Versuche, die Schmelze zu kristallisieren, führen in der Regel zu Mischungen von kristallinen und amorphen Anteilen. Die amorphen Anteile sind aber in höchstem Grade unerwünscht. Sie verursachen, selbst in geringen Mengen, eine Vergrünung oder Vergilbung des an sich farblosen Produktes unter dem Einfluss von Licht. So führt z. B. die Granulierung einer Schmelze/Kristall-Suspension auf einem Kühlband oder in einem Sprühturm immer zu einem teilweise amorphen und deshalb ungeeigneten Granulat.

Es bestand daher ein Bedürfnis, Pentaerythrit-tetrakis-[3-(3,5-di-tert.-butyl-4-hydroxyphenyl)-propionat] ohne Lösungsmittel direkt und vollständig in kristalline Form überzuführen, wobei ein solches Verfahren grosstechnisch anwendbar sein sollte. Es wurde nun gefunden, dass eine solche vollständige Kristallisation überraschenderweise direkt aus der Schmelze möglich ist, wenn letztere unter bestimmten Bedingungen in einem Extruder, einem Kneter oder einem Innenmischer mit bestimmten Kristallformen des Produktes angeimpft wird.

Die vorliegende Erfindung betrifft somit ein Verfahren zur vollständigen lösungsmittelfreien Kristallisation von Pentaerythrit-tetrakis-[3-(3,5-di-tert.-butyl-4-hydroxyphenyl)-propionat], das dadurch gekennzeichnet ist, dass man eine Schmelze dieser Verbindung in einem Extruder, Kneter oder Innenmischer mit mindestens 0,1-5 Gew.-%, bezogen auf die Schmelze, der β-, δ- oder λ-Kristallmodifikation der genannten Verbindung bei einer Temperatur von 70 bis 130 °C animpft.

Dabei wird als Schmelze besonders zweckmässig die direkt bei der Umesterung von Methyl-3-(3,5-di-tert.-butyl-4-hydroxyphenyl)-propionat mit Pentaerythrit anfallende Schmelze eingesetzt.

Das erfindungsgemässe Verfahren wird in den in der Verfahrenstechnik hinreichend bekannten Apparaturen ausgeführt, die als Extruder, Kneter und Innenmischer bezeichnet werden. Bevorzugt ist die Verwendung eines Extruders, beispielsweise eines Ein- oder Doppelschneckenextruders oder Planetwalzenextruders. Wird ein Extruder oder Innenmischer eingesetzt, wird das Verfahren vorzugsweise kontinuierlich durchgeführt, während in einem Kneter zweckmässig batchwise gearbeitet wird.

Zum Animpfen können die bekannten β- oder δ-Kristallmodifikationen von Pentaerythrit-tetrakis-[3-(3,5-di-tert.-butyl-4-hydroxyphenyl)-propionat] verwendet werden, bevorzugt ist aber die Verwendung der weiter unten beschriebenen neuen λ-Modifikation. Dabei ergibt das erfindungsgemässe Verfahren beim Einsatz von Impfkristallen der β-Modifikation das Produkt ebenfalls in der β-Modifikation, beim Einsatz von Impfkristallen der δ- und λ-Modifikation wird das kristallisierte Produkt jeweils in der λ-Modifikation erhalten.

Bevorzugt werden die Impfkristalle in einer Menge, bezogen auf die Schmelze, von 0,1 bis 3, insbesondere 0,1 bis 2, z. B. 0,8-1,2, vor allem etwa 1 Gew.-% zugesetzt. Selbstverständlich können die Impfkristalle auch in einer Menge von mehr als 5 % ohne Qualitäts- oder Ausbeuteeinbusse am kristallisierten Produkt eingesetzt werden. Höhere Mengen sind jedoch unwirtschaftlich und werden daher in der Praxis nicht angewendet.

Besonders zu erwähnen ist die Ausführungsform, in der man die bei der Umesterung von Methyl-3-(3,5-di-tert.-butyl-4-hydroxyphenyl)-propionat mit Pentaerythrit anfallende Schmelze mit 0,5-5 Gew.% Pentaerythrit-tetrakis-[3-(3,5-di-tert.-butyl-4-hydroxyphenyl)-propionat] der λ-Kristallmodifikation, bezogen auf die Schmelze, bei 70-130 °C animpft.

Der bevorzugte Temperaturbereich, bei dem das erfindungsgemässe Verfahren ausgeführt wird, liegt zwischen 70 °C und der Schmelztemperatur der jeweils verwendeten Mischkristalle bzw. der Mischung aus Schmelze und Mischkristallen. Beispiele für praktisch anwendbare Temperaturbereiche reichen von 80 °C, insbesondere 90 °C, bis zum Schmelzpunkt der jeweiligen Mischkristalle. Es sollte jedenfalls eine Temperatur gewählt werden, bei der während der Verweilzeit in der Apparatur die Impfkristalle nicht vollständig schmelzen. Ein bevorzugter Temperaturbereich ist 90-110 °C, insbesondere 95-105 °C, z. B. 100 °C.

Die erfindungsgemässe Kristallisation der Schmelze kann sehr schnell und damit wirtschaftlich erfolgen. Bei Verwendung eines Extruders sind z. B. Verweilzeiten von 3-5 Minuten in der Praxis realisierbar.

Allgemein hängt die Verweilzeit (Kristallisationszeit) auch davon ab, ob batchwise oder kontinuierlich gearbeitet wird. Sie kann z. B. zwischen 1 und 30, vorzugsweise 2-20, insbesondere 2-10 Minuten liegen.

Durch das erfindungsgemässe Verfahren wird eine 100 %-ige Kristallisation der Schmelze erreicht, d. h. es entstehen keine amorphen Anteile. Das erhaltene Granulat entspricht hohen Qualitätsanforderungen, da es lichtstabil ist. Würde man das Animpfen der Schmelze mit den genannten Impfkristallen z. B. in einer Labor-Rührapparatur durchführen, käme es zwar ebenfalls zu einer Kristallisation der Schmelze, jedoch wäre eine Rührzeit von etwa 1 Stunde oder mehr nötig und das erhaltene Produkt enthielte immer noch mindestens 1-3 % amorphe Anteile und würde damit den in der Praxis gestellten Qualitätsanforderungen nicht genügen.

Die vorliegende Erfindung betrifft weiter eine neue Kristallmodifikation (λ-Modifikation) von Pentaerythrit-tetrakis-[3-(3,5-di-tert.-butyl-4-hydroxyphenyl)-propionat], gekennzeichnet, durch ein Röntgenstrahlenbeugungsdiagramm, welches bei einem Netzebenenabstand von 5,24, 8,4 und 12,4 Å Linien sehr starker Intensität, bei 4,62, 6,3, 6,6, 6,7, 7,2 und 13,3 Å Linien starker Intensität, bei 3,78, 3,99, 4,14, 4,22, 4,25, 4,35, 4,44, 4,74, 4,84, 4,98, 5,06, 5,63, 6,0, 9,2 und 11,7 Linien mittlerer Intensität und bei 3,38, 3,43, 3,72, 3,82, 4,03, 4,40, 4,79, 5,52, 7,0, 10,0, 12,2 und 17,7 Å Linien schwacher Intensität aufweist.

Es wurde gefunden, dass diese neue λ-Modifikation aus der aus dem US-Patent 4 405 807 bekannten thermodynamisch unstabilen δ-Modifikation durch thermische Behandlung erhalten werden kann.

Die vorliegende Erfindung betrifft daher auch ein Verfahren zur Herstellung der oben beschriebenen λ-Modifikation, das dadurch gekennzeichnet ist, dass man eine Schmelze von Pentaerythrit-tetrakis-[3-(3,5-di-tert.-butyl-4-hydroxyphenyl)-propionat], die δ-Kristalle dieser Verbindung enthält, unter guter Durchmischung auf eine Temperatur zwischen 90 °C und dem Schmelzpunkt des Systems Schmelze/δ-Kristalle erhitzt.

Die neue λ-Modifikation weist einen Schmelzpunkt von etwa 124 °C auf, gegenüber 118 °C der δ-Modifikation bzw. ca. 116 °C der β-Modifikation.

Die Konzentration an δ-Kristallen in der Schmelze ist nicht kritisch, sie kann z. B. die oben für das erfindungsgemässe Schmelzkristallisationsverfahren angegebenen Werte haben.

Der Temperaturbereich, in dem die Herstellung der neuen λ-Modifikation erfolgen kann, ist z. B. 90-115 °C, vorzugsweise 95-115 °C, vor allem 100-110 °C. Vorzugsweise wird bei der Umwandlungstemperatur gerührt, zweckmässigerweise, um eine möglichst weitgehende Umwandlung zu erhalten, für längere Zeit, z. B. 1/2 Stunde bis 10 Stunden, z. B. 1 bis 5 Stunden. Wird die Umwandlung in üblichen Laborapparaturen durchgeführt, ist die Umwandlung nicht vollständig, da amorphe Anteile entstehen (siehe oben). Dieses einfache Verfahren kann jedoch dazu benutzt werden, λ-Impfkristalle für die erfindungsgemässe Schmelzkristallisation zu erzeugen. Selbstverständlich können die λ-Kristalle auch direkt durch letztere erhalten werden, wenn darin Impfkristalle der δ-Modifikation eingesetzt werden. In der Praxis werden dann natürlich vom entstehenden λ-Kristallisat Impfkristalle in die Kristallisationsapparatur zurückgeführt. Man startet also z. B. mit δ-Impfkristallen und führt dann kontinuierlich einen Teil des erhaltenen (z. B. extrudierten) λ-Kristallisats als Impfkristalle in den Prozess zurück.

Die thermodynamisch stabile neue λ-Kristallmodifikation von Pentaerythrit-tetrakis-[3-(3,5-di-tert.-butyl-4-hydroxyphenyl)-propionat] ist eine bevorzugte Modifikation dieser Verbindung, die gegenüber anderen Modifikationen verschiedene Vorteile aufweist. So ist z. B. die Verwendung von λ-Kristallen als Impfkristalle im erfindungsgemässen Schmelzkristallisationsverfahren besonders bevorzugt, da mit diesen die Kristallisation besonders schnell und vollständig abläuft. Ausserdem weist die λ-Modifikation einen noch höheren Schmelzpunkt als die bekannte β- und δ-Modifikation auf, was einen Vorteil hinsichtlich Transport- und Lagerstabilität bedeutet.

Die folgenden Beispiele dienen der weiteren Erläuterung der Erfindung, ohne diese einzuschränken.

Beispiel 1 : Herstellung von λ-Impfkristallen

In einem Kolben werden 1 000 g Pentaerythrit-tetrakis-[3-(3,5-di-tert.-butyl-4-hydroxyphenyl)-propionat] (δ-Form, Smp. 114-116 °C) auf 130 °C erhitzt, wobei eine klare Schmelze entsteht. Diese wird auf 105 °C abgekühlt und mit 10 g kristallinem Pentaerythrit-tetrakis-[3-(3,5-di-tert.-butyl-4-

hydroxyphenyl)-propionat] (δ-Form) versetzt. Die Suspension wird bei ca. 105 °C weiter gerührt. Nach 2 bis 3 Stunden setzt die Kristallisation ein. Die Innentemperatur steigt an und muss durch Aussenkühlung unterhalb 115 °C gehalten werden. Die Suspension wird zunehmend dicker und erstarrt schliesslich zu einer harten Kristallmasse. Diese wird nach dem Abkühlen zerkleinert. Nach Röntgenstrahlenanalyse handelt es sich um Kristalle der λ-Modifikation. Die Differentialthermoanalyse zeigt neben einem Hauptanteil mit Smp. 122-123 °C (λ) noch 1 bis 3 % amorphes Produkt mit Smp. 55 bis 60 °C.

Beispiel 2 : Kristallisation der Schmelze durch Animpfen mit λ-Impfkristallen

Aus einem Vorratsgefäss wird geschmolzenes Pentaerythrit-tetrakis-[3-(3,5-di-tert.-butyl-4-hydroxyphenyl)-propionat] bei einer Temperatur von 100-105 °C einem Pilot-Planetwalzenextruder (ohne Düse) mit einer Geschwindigkeit von 5 kg/h bei einer Schneckendrehzahl von 20 U/Min. zugeführt. Der Extruder wird gleichmässig auf 90 °C beheizt. Gleichzeitig werden mittels eine Dosierschnecke Kristalle der λ-Modifikation von Pentaerythrit-tetrakis-[3-(3,5-di-tert.-butyl-4-hydroxyphenyl)-propionat] (wie sie z. B. nach der in Beispiel 1 beschriebenen Methode erhalten werden) mit einer Geschwindigkeit von ca. 0,05 kg/h (entsprechend einer Konzentration von ca. 1 Gew.-%, bezogen auf die Schmelze) zu der im Extruder beförderten Schmelze zudosiert. Die Produkttemperatur im Extruder steigt auf ein Maximum von 110 °C, sinkt dann aber wieder. Das in Form von groben, harten Brocken anfallende Produkt wird nachträglich zerkleinert. Es besteht einheitlich aus Kristallen der λ-Modifikation. Die Differentialthermoanalyse zeigt, dass keine amorphen Anteile vorhanden sind. Smp. 124 °C.

Im weiteren Verlauf der Extruder-Kristallisation wird dann selbstverständlich ein Teil dieser Ware als Impfsubstanz in den Prozess zurückgeführt. In der Praxis wird als Schmelze die direkt aus der Umesterung von Methyl-3-(3,5-di-tert.-butyl-4-hydroxyphenyl)-propionat mit Pentaerythrit anfallende Produktschmelze eingesetzt.

Beispiel 3

Beispiel 2 wird wiederholt, wobei aber an Stelle von 0,05 kg/h der λ-Modifikation 0,065 kg/h von Kristallen der δ-Modifikation von Pentaerythrit-tetrakis-[3-(3,5-di-tert.-butyl-4-hydroxyphenyl)-propionat] mit einem Smp. von 112-118 °C (entsprechend einer Konzentration von ca. 1,3 Gew.-%, bezogen auf die Schmelze) mittels einer Dosierschnecke zugeführt werden. Die Temperatur im Extruder steigt dann auf ein Maximum von 114 °C. Es entsteht ebenfalls ein einheitliches Produkt aus λ-Kristallen mit einem Smp. von 124 °C.

Beispiel 4

Beispiel 2 wird wiederholt, wobei aber an Stelle von 0,05 kg/h der λ-Modifikation 0,035 kg/h von Kristallen der β-Modifikation von Pentaerythrit-tetrakis-[3-(3,5-di-tert.-butyl-4-hydroxyphenyl)-propionat] mit einem Smp. von 111-116 °C (entsprechend einer Konzentration von ca. 0,7 Gew.-%, bezogen auf die Schmelze) mittels einer Dosierschnecke zugeführt werden. Die Temperatur im Extruder steigt dann auf ein Maximum von 105 °C. Es entsteht ebenfalls ein einheitliches Produkt aus β-Kristallen (ohne amorphe Anteile) mit einem Smp. von 116 °C.

Patentansprüche

1. Verfahren zur vollständigen lösungsmittelfreien Kristallisation von Pentaerythrit-tetrakis-[3-(3,5-di-tert.-butyl-4-hydroxyphenyl)-propionat], dadurch gekennzeichnet, dass man eine Schmelze dieser Verbindung in einem Extruder, Kneter oder Innenmischer mit mindestens 0,1-5 Gew.-%, bezogen auf die Schmelze, der β-, δ- oder λ-Kristallmodifikation der genannten Verbindung bei einer Temperatur von 70 bis 130 °C animpft.

2. Verfahren nach Anspruch 1, worin als Schmelze die bei der Umesterung von Methyl-3-(3,5-di-tert.-butyl-4-hydroxyphenyl)-propionat mit Pentaerythrit anfallende Schmelze eingesetzt wird.

3. Verfahren nach Anspruch 1, dadurch gekennzeichnet, dass es in einem Extruder durchgeführt wird.

4. Verfahren nach Anspruch 3, dadurch gekennzeichnet, dass es kontinuierlich durchgeführt wird.

5. Verfahren nach Anspruch 1, dadurch gekennzeichnet, dass mit Kristallen der λ-Form angeimpft wird.

6. Verfahren nach Anspruch 1, dadurch gekennzeichnet, dass man die Impfkristalle in einer Menge von 0,1 bis 2, vorzugsweise 0,8 bis 1,2 Gew.-%, bezogen auf die Schmelze, einsetzt.

7. Verfahren nach Anspruch 1, dadurch gekennzeichnet, dass man die bei der Umesterung von Methyl-3-(3,5-di-tert.-butyl-4-hydroxyphenyl)-propionat mit Pentaerythrit anfallende Schmelze mit 0,5-5 Gew.-% Pentaerythrit-tetrakis-[3-(3,5-di-tert.-butyl-4-hydroxyphenyl)-propionat] der λ-Kristallmodifikation, bezogen auf die Schmelze, bei 70-130 °C animpft.

8. Verfahren nach Anspruch 1, dadurch gekennzeichnet, dass man bei einer Temperatur von 70 °C bis zur Schmelztemperatur der jeweiligen Impfkristalle arbeitet.

9. Verfahren nach Anspruch 8, dadurch gekennzeichnet, dass man bei einer Temperatur von 90-110 °C arbeitet.

10. λ-Modifikation von Pentaerythrit-tetrakis-[3-(3,5-di-tert.-butyl-4-hydroxyphenyl)-propionat], gekennzeichnet durch ein Röntgenstrahlenbeugungsdiagramm, welches bei einem Netzebenabstand von 5,24, 8,4 und 12,4 Å Linien sehr starker Intensität, bei 4,62, 6,3, 6,6, 6,7, 7,2 und 13,3 Å Linien starker Intensität, bei 3,78, 3,99, 4,14, 4,22, 4,25, 4,35, 4,44, 4,74, 4,84, 4,98, 5,06, 5,63, 6,0, 9,2, und 11,7 Linien mittlerer Intensität und bei 3,38, 3,43, 3,72, 3,82, 4,03, 4,40, 4,79, 5,52, 7,0, 10,0, 12,2 und 17,7 Å Linien schwacher Intensität aufweist.

11. Verfahren zur Herstellung der λ-Modifikation gemäss Anspruch 10, dadurch gekennzeichnet, dass man eine Schmelze von Pentaerythrittetrakis-[3-(3,5-di-tert.-butyl-4-hydroxyphenyl)-propionat], die δ-Kristalle dieser Verbindung enthält, unter guter Durchmischung auf eine Temperatur zwischen 90 °C bis zum Schmelzpunkt des Systems Schmelze/δ-Kristalle erhitzt.

## Claims

1. A process for the complete, solvent-free crystallisation of penta-erythritol tetrakis-[3-(3,5-di-tert-butyl-4-hydroxyphenyl)propionate], which comprises seeding a melt of this compound in an extruder, a kneader or an internal mixer with at least 0.1 to 5 % by weight, based on the melt, on the β-, δ- or λ-crystal modification of the stated compound, at a temperature from 70 to 130 °C.

2. A process according to claim 1, in which the melt obtained from the transesterification of methyl 3-(3,5-di-tert-butyl-4-hydroxyphenyl)propionate with pentaerythritol is used as the melt.

3. A process according to claim 1 which is carried out in an extruder.

4. A process according to claim 3 which is carried out continuously.

5. A process according to claim 1, wherein the melt is seeded with crystals of the λ-modification.

6. A process according to claim 1, wherein the seed crystals are used in an amount of 0.1 to 2, preferably of 0.8 to 1.2 % by weight, based on the melt.

7. A process according to claim 1, which comprises seeding the melt obtained from the transesterification of methyl 3-(3,5-di-tert-butyl-4-hydroxyphenyl)propionate with pentaerythritol at 70-130 °C with 0.5 to 5 % by weight, based on the melt, of the λ-crystal modification of pentaerythritol tetrakis-[3-(3,5-di-tert-butyl-4-hydroxyphenyl)propionate].

8. A process according to claim 1, wherein crystallisation is carried out at a temperature from 70 °C to the melt temperature of the respective seed crystals.

9. A process according to claim 8, wherein crystallisation is carried out at a temperature from 90-110 °C.

10. The λ-modification of pentaerythritol tetrakis-[3-(3,5-di-tert-butyl-4-hydroxyphenyl)propionate], characterised by an X-ray diffraction pattern which exhibits lines of very high intensity at an interplanar spacing of 5.24, 8.4 and 12.4 Å, lines of strong intensity at an interplanar spacing of 4.62, 6.3, 6.6, 6.7, 7.2 and 13.3 Å, lines of medium intensity at an interplanar spacing of 3.78, 3.99, 4.14, 4.22, 4.25, 4.35, 4.44, 4.74, 4.84, 4.98, 5.06, 5.63, 6.0, 9.2 and 11.7, and lines of weak intensity at an interplanar spacing of 3.38, 3.43, 3.72, 3.82, 4.03, 4.40, 4.79, 5.52, 7.0, 10.0, 12.2 and 17.7 Å.

11. A process for the preparation of the λ-modification according to claim 10, which comprises heating a melt of pentaerythritol tetrakis-[3-(3,5-di-tert-butyl-4-hydroxyphenyl)propionate] which contains δ-crystals of this compound, while mixing thoroughly, to a temperature in the range from 90 °C to the melting point of the system consisting of melt/δ-crystals.

## Revendications

1. Procédé pour faire cristalliser complètement sans solvant le tétrakis-[(di-tert-butyl-3,5 hydroxy-4 phényl)-3 propionate] du pentaérythrol, procédé caractérisé en ce qu'on ensemence, à une température de 70 à 130 °C, une masse fondue de ce composé dans une extrudeuse, un malaxeur ou un mélangeur interne avec au moins 0,1 à 5 % en poids, par rapport à la masse fondue, d'une des modifications cristallines β, δ et λ du composé cité.

2. Procédé selon la revendication 1 dans lequel on utilise, comme masse fondue, celle qui est obtenue lors de la trans-estérification du (di-tert-butyl-3,5 hydroxy-4 phényl)-3 propionate de méthyle avec le pentaérythrol.

3. Procédé selon la revendication 1, caractérisé en ce qu'il est effectué dans une extrudeuse.

4. Procédé selon la revendication 3, caractérisé en ce qu'il est effectué en continu.

5. Procédé selon la revendication 1, caractérisé en ce qu'on ensemence avec des cristaux de la forme λ.

6. Procédé selon la revendication 1, caractérisé en ce que les germes (cristaux d'ensemencement) sont mis en jeu en une quantité de 0,1 à 2 % en poids par rapport à la masse fondue, de préférence en une quantité de 0,8 à 1,2 % en poids.

7. Procédé selon la revendication 1, caractérisé en ce qu'on ensemence la masse fondue obtenue lors de la transestérification du (di-tert-butyl-3,5 hydroxy-4 phényl)-3 propionate de méthyle avec le pentaérythrol, à une température de 70 à 130 °C, avec de 0,5 à 5 % en poids de tétrakis-[(di-tert-butyl-3,5 hydroxy-4 phényl)-3 propionate] du pentaérythrol de la modification λ, par rapport à la masse fondue.

8. Procédé selon la revendication 1, caractérisé en ce qu'on opère à une température comprise entre 70 °C et la température de fusion des cristaux germes utilisés.

9. Procédé selon la revendication 8, caractérisé en ce qu'on opère à une température de 90 à 110 °C.

10. Modification λ du tétrakis-[(di-tert-butyl-3,5 hydroxy-4 phényl)-3 propionate] du pentaérythrol, caractérisée en ce que son diagramme de diffraction des rayons X présente des raies très intenses pour des distances entre plans réticulaires de 5,24, de 8,4 et 12,4 Å, des raies de forte intensité pour des distances de 4,62, de 6,3, de 6,6, de 6,7, de 7,2 et de 13,3 Å, des raies d'intensité moyenne pour des distances de 3,78, de 3,99, de 4,14, de 4,22, de 4,25, de 4,35, de 4,44, de 4,74, de 4,84, de 4,98, de 5,06, de 5,63, de 6,0, de 9,2 et de 11,7, et des raies de faible intensité pour des distances de 3,38, de 3,43, de 3,72, de 3,82, de 4,03, de 4,40, de 4,79, de 5,52, de 7,0, de 10,0, de 12,2 et de 17,7 Å.

11. Procédé de préparation de la modification λ selon la revendication 10, procédé caractérisé en ce qu'on chauffe une masse fondue de tétrakis-[(di-tert-butyl-3,5 hydroxy-4 phényl)-3 propionate] du pentaérythrol contenant des cristaux δ de ce composé, tout en mélangeant bien, à une température comprise entre 90 °C et le point de fusion du système constitué par la masse fondue et les cristaux δ.